# EUROPEAN PATENT SPECIFICATION

(11) **EP 2 520 306 B1**
(45) Date of publication and mention of the grant of the patent: **06.12.2017**
(21) Application number: 11425125.9
(22) Date of filing: 05.05.2011
(51) Int. Cl.: A61K 36/28, A61K 8/97, A61P 29/00, A61P 17/00, A61P 17/02

(54) **Process for preparing an inflorescence extract of Calendula Officinalis by means of extraction with supercritical carbon dioxide and compositions containing said extract**
Verfahren zur Herstellung eines Infloreszenzextrakts von Calendula officinalis mittels Extraktion mit überkritischem Kohlendioxid und Zusammensetzungen, die diesen Extrakt enthalten
Procédé pour la préparation d'un extrait d'inflorescence de Calendula officinalis au moyen de l'extraction avec un dioxyde de carbone supercritique et compositions contenant ledit extrait

(43) Date of publication of application: 07.11.2012
(73) Proprietor: Unifarco S.p.A., 32035 Santa Giustina (BL) (IT)
(72) Inventor: Baratto, Giovanni, 32035 Santa Giustina (BL) (IT); Riva, Ernesto, 32035 Santa Giustina (BL) (IT)
(74) Representative: Asensio, Raffaella Consuelo

(56) References cited:
- WO-A1-03/037833
- DELLA LOGGIA R ET AL: "THE ROLE OF TRITERPENOIDS IN THE TOPICAL ANTI-INFLAMMATORY ACTIVITYOF CALENDULA OFFICINALIS FLOWERS", PLANTA MEDICA, THIEME VERLAG, DE, vol. 60, no. 6, 1 January 1994 (1994-01-01), pages 516-520, XP000604247, ISSN: 0032-0943
- FUCHS S.M. ET AL.: "Protective effects of different marigold (Calendula officinalis L.) and rosemary cream preparations against sodium-lauryl-sulfate-induced irritant contact dermatitis", SKIN PHARMACOLOGY AND PHYSIOLOGY, vol. 18, no. 4, 2005, pages 195-200, XP009151770,
- REZNICEK GOTTFRIED, ZITTERL-EGLSEER KARIN: "Quantitative determination of the faradiol esters in marigold flowers and extracts", SCIENTIA PHARMACEUTICA 20030630 AT, vol. 71, no. 2, 30 June 2003 (2003-06-30), pages 121-128, XP009151772, ISSN: 0036-8709
- REZNICEK G ET AL: "Analytik der Faradiolmonoester in Ringelblumenblüten, Extrakten und Salben = Analysis of the faradiol esters in marigold, extracts and ointments", ZEITSCHRIFT FUER PHYTOTHERAPIE, HIPPOKRATES VERLAG IN MVS MEDIZINVERLAGE, DE, vol. 21, no. 3, 1 January 2000 (2000-01-01), pages 152-153, XP009151769, ISSN: 0722-348X
- PREETHI K C ET AL: "Anti-inflammatory and wound healing activity of Calendula officinalis", MEDICINAL & AROMATIC PLANTS ABSTRACTS, SCIENTIFIC PUBLISHERS, SCIENTIFIC PUBLISHERS, NEW DELHI - INDIA, vol. 29, no. 6, 1 December 2007 (2007-12-01), XP018022683, ISSN: 0250-4367

## Description

### Field of the invention

The present invention regards a process for preparing an extract of *Calendula officinalis* and cosmetic and pharmaceutical compositions that contain it.

### Background of the invention

*Calendula officinalis* is a plant belonging to the Asteraceae family, known for its numerous phytotherapeutic properties, mainly for its anti-inflammatory, lenitive and cicatrizing properties. The drug is constituted by the flowers and by the flowering tops, from which tinctures and extracts are obtained that are usually used for preparing topical formulations. Among the active principles contained in the drug, an important role is carried out by the esters of triterpene alcohols such as calenduladiol, arnidiol and faradiol.

The extracts of *Calendula officinalis* obtained by means of extraction with organic solvent generally have a low, non-standardized content of such esters and also contain other substances unnecessary for obtaining the desired effect; therefore, considerable amounts of extract must be used for the preparation of the finished products, with clear difficulties from the formulation standpoint. In addition, it is necessary to make sure that there are no solvent residues in the finished products, in order to avoid allergic reactions; finally, it is always preferable to avoid the use of organic solvents in order to reduce environmental impact.

It is known that plant extracts can be prepared by means of extraction with carbon dioxide in supercritical phase, which generally allows maintaining the active "principles contained in the plant material integral, in particular those more unstable; this method was also used for the preparation of *Calendula officinalis* extracts. For example, Hamburger M. et al, Fitoterapia 74, 328-338 (2003) describe the purification of the triterpenoid esters of calendula by means of a combination of extraction with carbon dioxide in supercritical phase and normal phase and reversed phase column chromatography; the extraction with carbon dioxide in supercritical phase is carried out at the pressure of 500 bar and at the temperature of 50°C, with a carbon dioxide flow speed of 35 kg h-1. At the end of the process, an extract is obtained that contains the triterpenoid mixture in amounts of 18-20% wherein the faradiol ester amount is of about 85% faradiol total esters; nevertheless, the extract must be subjected to further purification stages in order to eliminate undesired components, particularly the carotenoids.

Baumann D. et al, Phytochemical analysis 15, 226-230 (2004), analytically describe the extraction on a pilot scale of calendula flowers via carbon dioxide in supercritical phase, at a pressure variable between 300 and 800 bar and at the temperature of 50°C. The authors observe that a qualitative and quantitative improvement in the sole faradiol myristate can be obtained by increasing the pressure and by adding a small amount of an extraction modifier, i.e. 0.5% ethanol.

Danielski L. et al, Chemical Engineering and Processing 46, 99-106 (2007), describe the preparation of an oleoresin from calendula flowers coming from Brazil by means of extraction with carbon dioxide in supercritical phase at a temperature comprised between 20.0 and 40.0°C and a pressure comprised between 120.0 and 200.0 bar. Due to the origin of the plant and the extraction conditions, triterpenoid esters, in particular faradiol esters, are not present in the oleoresin.

Della Loggia R et al." The role of triterpenoids in the topical anti-inflammatory activity of calendula officinalis flowers" Planta Medica,, Theme Verlag DE, vol.60, 1.01.1994, pages 515-520*)* disclose a CO2 extract containing a mixture of the triterpene ester (calenduladiol, arnidiol and faradiol esters) in amounts of about 25% wherein the faradiol esters represent in total 75% of the above triterpene mixture, wherein the most active compounds,i.e. the faradiol esters are present in amount of 19%.

Fuchs et al : "Protective effects of different marigold extracts of different marigold (Calendula Officinalis L.) and rosemary cream preparations against sodium laurysulfate indiced irritant contact dermatitis" Skin Pharmacology and Physiology, vol.18, no.4 , 2005, pages 195-200) report that dyed and undyed CO2 extracts of marigold are effective in the treatment of irritant contact dermatitis. However in this reference nothing is said about the composition of said CO2 extracts and moreover nothing is said about the process for preparing these extracts.

Reznicek et al.: "Quantitative determination of the faradiol esters in marigold flowers and extracts" Scientia Pharmaceutica 20030630AT, vol.71, no.2 30 June 2003 pages 121-128, disclose an extract containing faradiol esters in amounts of 29.82%. and the same authors in Analitik der Faradiolmonoester in Ringelblumenblueten, Extracten und Salben" Zeitschrift fuer Phytotherapie, Hippocrates Verlag in MVS Medizineverlage, DE, vol.21, No. 3 , 1 January 2000 pages 152-153, report that faradiol esters reach 20-30% in CO2 extracts WO03/037833 discloses a process of extraction with CO2 from meals of marigold petals, it is directed to the extraction of a chemical compound namely lutein diester.

### Description of the invention

It has now been found that by subjecting flowers of *Calendula officinalis* to extraction with carbon dioxide in supercritical phase at specific conditions, it is possible to obtain products with different consistency. These products, when mixed together, form a soft extract having a standardized and balanced content of triterpene esters, and it was found that this extract can be conveniently used for preparing topical lipophilic compositions having lenitive, protective and anti-reddening action. The present invention therefore relates to:
- a process for extracting triterpene ester mixture from flowering tops of *Calendula officinalis* as claimed in claim 1-2, a process for preparing total extracts as claimed in claims 3-4,
- the total extracts claimed in claims 5 and 6,
- the use as a medicament of the total extract, as claimed in claim 7, wherein said medicament is in the form of a topical formulation containing said total extract in an amount comprised between 0.01 and 6% by weight with respect to the overall weight of said formulation,
- topical pharmaceutical or cosmetic formulation -containing this total extract in an amount comprised between 0.01 and 6% by weight with respect to the overall weight of said formulation as claimed in claim 8.,
- the use of the total extract of flowering tops according to claim 6) in cosmetic formulations, wherein said total extract is contained in said topical cosmetic formulation in an amount comprised between 0.01 and 6% by weight with respect to the overall weight of said formulation.

The process that is the subject of the present invention comprises the following stages:
a) providing flowering tops of *Calendula officinalis*;
b) subjecting said flowering tops to extraction with carbon dioxide in supercritical phase at a temperature comprised between 60 and 70°C and at a pressure comprised between 600 and 700 bar, for a period of time comprised between 200 and 250 min, preferably for 230 min;
c) decreasing the pressure to 200-300 bar and collecting the extract thus obtained (hereinbelow defined "extract A");
d) further decreasing the pressure to 60-70 bar and collecting the extract thus obtained (hereinbelow defined "extract B").

More in detail, dried flowering tops of *Calendula Officinalis* with a content of residual humidity comprised between 3 and 5 % w/w are ground and suitably sieved; the grinding can be carried out by means and techniques known to the man skilled in the art, for example by means of a hammer mill. The fraction having grain size comprised between 2 and 5 mm is placed in the extraction vessel and treated at the above-indicated conditions. Preferably, flowers of European origin are used.

The extraction process according to the invention can be carried out with conventional instruments; nevertheless, the extraction plant shall comprise, in addition to a first extraction chamber in which stage b) is carried out, also two heat exchangers and two collection chambers downstream of the extraction chamber for the separation of the extracts obtained in stages c) and d). An extraction plant that is particularly suitable for carrying out the process is described in the patent application DE 10 2009 023 549 A1.

The extracts A and B obtained with the process according to the invention are mixed in a weight ratio comprised between 0.5:9.5 (A:B) and 3:7 (A:B), preferably in a ratio of 1:9 (A:B); the resulting mixture, defined hereinbelow as the "total extract", can be conveniently used for preparing topical formulations for pharmaceutical or cosmetic use having lenitive, protective and anti-reddening action.

In the extracts A and B obtained with the process according to the invention, the ester content of the main triterpene alcohols, i.e. calenduladiol, amidiol and faradiol, in particular of the palmitic, lauric and myristic esters, ranges from 2 to 8% by weight; therefore, these extracts are considered enriched with triterpene esters. Consequently, the total extract obtained from their mixture contains at least 3% by weight of triterpene esters, expressed as a mixture of calenduladiol, amidiol and faradiol myristates, laurates and palmitates; the faradiol esters represent at least 35% by weight of the total triterpene esters. By virtue of the significant amount of triterpene esters, the topical formulations containing the extract ensure an effectiveness that is equal to or greater than that of the products heretofore known, and are highly tolerable due to the reduction of the components with undesired action.

Therefore, subject of the present invention are also topical formulations containing the total extract constituted by the extracts A and B in a weight ratio ranging from 0.5:9.5 (A:B) to 3:7 (A:B), preferably 1:9 (A:B), in a mixture with suitable excipients and/or carriers and possible further active ingredients. The total extract is contained in an amount comprised between 0.01% and 6% by weight with respect to the overall weight of the formulation. In addition, the formulations preferably have lipophilic matrix, such as lipogels and oleolites, ointments and emulsions.

The formulations according to the invention can be prepared with methods and ingredients that are known to the man skilled in the art, for example those described in Remington, The Science & Practice of Pharmacy, 21st edition.

In accordance with a preferred embodiment of the invention, the formulation is a lipogel containing the following ingredients, in the percentages by weight indicated herein below:

| | |
|---|---|
| Dimethicone | 28 - 34 % |
| Tocopheryl acetate | 18 - 26 % |
| Caprylyl methicone | 16 - 22 % |
| Hydrogenated castor oil | 6 - 10 % |
| Dicaprylyl carbonate | 6 - 9 % |
| Ethylhexyl stearate | 5 - 7 % |
| Palmitamide MEA | 0.5 - 7 % |
| Vegetable oil | 1 - 6 % |
| Total extract of *Calendula officinalis* according to the invention | 0.01 - 6 % |
| Preserving/antioxidant system | q.s. to 100 |

In accordance with a further preferred embodiment of the invention, the lipophilic formulation is an oleolite containing the following ingredients, in the percentages by weight indicated to the side:

| | |
|---|---|
| Cyclopentasiloxane | 70 - 80 % |
| Tocopheryl acetate | 18 - 26 % |
| Total extract of *Calendula officinalis* according to the invention | 0.01 - 6 % |
| Vegetable oil | 0.1 - 1 % |
| Ascorbyl tetraisopalmitate | 0.001 - 0.5 |
| Preserving/antioxidant system | q.s. to 100 |

In accordance with a further preferred embodiment of the invention, the formulation is an oil containing the following ingredients, in the percentages by weight indicated herein below:

| | |
|---|---|
| Dipentaerythrityl pentaisononanoate | 50 - 60 % |
| Tocopheryl acetate | 15 - 30 % |
| Hydrogenated polyisobutene | 5 - 20 % |
| Ascorbyl tetraisopalmitate | 0.02 - 0.5 % |
| Bisabolol | 0.05 - 0.5 % |
| Total extract of *Calendula officinalis* according to the invention | 0.1 - 5 % |
| Tocotrienols | 0.02 - 0.1 % |

Due to the balanced content of oils and waxes, the lipogel, the oleolite and the oil described above are stable against syneresis phenomena typical of lipophilic matrices; in addition, they optimize the effectiveness of the total extract and ensure high tolerability, while maintaining a suitable pleasantness in application (spreadability, absorption, dry residue without oiliness sensation).

The invention will now be described in more detail in the experimental part and in the examples below.

### EXPERIMENTAL PART

### Materials and methods

### Plant material

As raw material, flowering tops of *Calendula officinalis* were used that were cultivated in Valbelluna (BL) territory. The fully-flowered tops collected were dried at a constant temperature of 40°C until an average humidity was obtained of less than 5%. Before being subjected to extraction, the flowering tops were ground with a hammer mill.

### Equipment

The extraction with supercritical carbon dioxide was carried out in an industrial extraction plant as described in the patent application DE 10 2009 023 549 A1, which comprises an extraction chamber, two heat exchangers and two collection chambers downstream of the exchangers. The equipment for the qualitative-quantitative analysis of the extraction products and for the pharmacological assays is indicated below.

### Examples

### Example 1 - Extraction of flowering tops of Calendula officinalis with carbon dioxide in supercritical phase

170 kg of dried flowering tops of *Calendula officinalis* were subjected to extraction with carbon dioxide, operating as follows. The ground material having grain size comprised between 2 and 5 mm was loaded into an extraction chamber and subjected to a pressure comprised between 600 and 700 bar, maintaining the temperature in a range comprised between 60 and 70°C; the extraction time was 230 minutes. The extraction fluid pressure was then reduced to 200 - 300 bar by means of the action of a heat exchanger; in this manner, it was possible to collect the extract A in the first condensation chamber. The pressure of the uncondensed fluid in this first chamber was further reduced to 60-70 bar, obtaining the separation of a second fraction (extract B) collected in the second condensation chamber. A total extraction yield was obtained equal to 8% by weight of the total dry drug, in particular 1% extract A and 7% extract B.

The extracts A and B were mixed with a Comer® mixer in a 1:9 weight ratio in order to obtain the total extract used in the preparation of the lipogel and the oleolite described in examples 2 and 3.

### Example 2 - Lipogel containing the total extract of Calendula officinalis obtained according to example 1

100 g of lipogel were prepared with the following ingredients in the following amounts:

| | |
|---|---|
| Dimethicone (DC200 Fluid, Dow Corning) | 27 g |
| Tocopheryl acetate (dl alpha tocopheryl acetate, Respharma) | 24 g |
| Caprylyl methicone (Dow Corning, Toray FZ - 3196) | 18.00 g |
| Hydrogenated castor oil (Cutina HR, Cognis) | 9.00 g |
| Dicaprylyl carbonate (Cetiol CC, Cognis) | 6.00 g |
| Ethylhexyl stearate (Cetiol 868, Cognis) | 6.00 g |
| Palmitamide MEA (PMEA, Nikkol) | 7.00 g |
| Vegetable oil (Cegesoft PS17, Cognis) | 1.00 g |
| Total extract of *Calendula officinalis* according to example 1 | 1.50 g |
| Preserving/antioxidant system | 0.5 g |

### Preparation

The suitably-weighed oily phase (dimethicone, caprylyl methicone, hydrogenated castor oil, dicaprylyl carbonate, ethylhexyl stearate, vegetable oil) was introduced in a Comer® turboemulsifier. The load door was closed and a vacuum (-0.7 atm) was applied to the mixing tank. The oily mass was maintained under stirring, with one blade rotating and the other counter-rotating, at the speed of 60 rpm and at a temperature of 75-80°C, in order to allow the waxes present to suitably melt. The temperature was then decreased to 65-70°C, the vacuum was removed and the following were added: palmitamide MEA, tocopheryl acetate, total calendula extract obtained in example 1 and antioxidants, under constant stirring, after which a vacuum was once again applied (-0.7 atm). The product was cooled in a gradual manner, proceeding at intervals of about 5-8°C, interrupted by a turboemulsifying step, driving the turbine at 1480 rpm for 1-3 minutes. Once the temperature of 45-50°C was reached, the product was gradually cooled, avoiding marked sudden changes, mixture was maintained under stirring until room temperature, thereafter the vacuum is removed and the product unloaded into a suitable container.

### Example 3 - Oleolite containing the extract of Calendula officinalis obtained according to example 1

100 g of oleolite were prepared with the following ingredients in the following amounts:

| | |
|---|---|
| Cyclopentasiloxane (DC 245 Fluid, Dow Corning) | 78.00 g |
| Tocopheryl acetate (dl alpha tocopheryl acetate) | 20.00 g |
| Vegetable oil (Cegesoft PS17, Cognis) | 0.70 g |
| Bisabolol (natural alpha bisabolol) | 0.10 g |
| Ascorbyl tetraisopalmitate (Nikkol VC-IP) | 0.10 g |
| Total extract of *Calendula officinalis* according to example 1 | 1.10 g |

### Preparation

The oily phase (cyclopentasiloxane, vegetable oil), suitably weighed, was introduced into a Comer blade mixer under stirring at 60 rpm and at room temperature. Once a homogeneous solution had been achieved, the following were added under constant stirring: ascorbyl tetraisopalmitate, tocopheryl acetate, total calendula extract obtained according to example 1 and antioxidants. Once a homogeneous product had been obtained, the stirring was terminated and the product unloaded into a suitable container.

### Example 4 - Oil containing the extract of Calendula officinalis obtained according to example 1

| | |
|---|---|
| Tocopheryl acetate (dl alpha tocopheryl acetate, Respharma) | 22.00 g |
| Ascorbyl tetraisopalmitate (Nikkol VC-IP, Nikko) | 0.10 g |
| Hydrogenated polyisobutene (MC 300) | 19.20 g |
| Dipentaerythrityl pentaisononanoate (DUB Vinyl) | 57.00g |
| Tocotrienols (Mixed Tocotrienols 92) | 0.10 g |
| Bisabolol (natural alpha bisabolol) | 0.10 g |
| Total extract of *Calendula officinalis* according to example 1 | 1.50 g |

### Preparation

The ingredients were introduced in a mixer one at a time, under cold conditions; mixing took place until a homogeneous product was obtained.

### Qualitative-quantitative analysis of the triterpene esters in the extracts obtained according to example 1

The extracts A, B and the total extract obtained according to example 1 were subjected to qualitative-quantitative analysis by using HPLC-APCI-MS and HPLC-DAD.

### Materials and methods

The following were used: an Agilent 1100 chromatograph equipped with series 1100 diode detector, and a HPLC Varian series 212 chromatographic system equipped with a Varian MS500 ionic trap detector, equipped with APCI (Atmospheric Pressure Chemical Ionization) source. As stationary phase, a Waters X-terra 3.5 x 150 (3.5 micron) column was used. As mobile phase, methanol was used, via isocrotic elution. The parameters of the mass spectrometer are reported in Table 1.

**Table 1**

| **Parameter** | **Value** |
|---|---|
| Temperature of the chamber | 50°C |
| Spray shield voltage | 600 V |
| Pressure of the nebulizer gas | 60 psi |
| Pressure of the drying gas | 20 psi |
| Temperature of the drying gas | 150°C |
| Capillary voltage | 150 V |
| RF load | 70 % |
| Crown current | 5 µA |

For the identification of the substances present in the extracts, the mass spectra and relative fragments were used; in HPLC-APCI-MS, the triterpene esters were present as groups of ions having m/z characteristics, in particular laurates ([M-H₂O+H]⁺ 607 ions), myristates ([M-H₂O+H]⁺ 635 ions) and palmitates ([M-H₂O+H]⁺ 663 ions). For such compounds, MS/MS measurements were also made.

In addition, the main compounds present in the extracts A and B were isolated by means of semi-preparative HPLC [Hamburger M. et al, Fitoterapia 74, 328-338 (2003)] and their structures were confirmed by means of NMR analysis. As reference substance for the quantitative analyses, faradiol-3-O-myristate was used, isolated from the extracts as described in Hamburger M. et al, Fitoterapia 74, 328-338 (2003). In order to construct the calibration line, solutions of faradiol-3-O-myristate were prepared with a concentration comprised between 12.7 µg/ml and 0.127 µg/ml.

It was observed that extracts A and B contain derivatives of the terpene alcohols calenduladiol, arnidiol and faradiol esterified with myristic, palmitic and lauric acid. As a standard for the quantitative analyses, faradiol myristate was used that was previously isolated by means of semi-preparative HPLC as described in Hamburger M. et al, Fitoterapia 74, 328-338 (2003). Table 2 reports the percentage amount of triterpenes in the extracts A and B obtained according to example 1 and in the total extract obtained by the mixing thereof.

**Table 2**

| LAURATES | | % in extract A | % in extract B | % in the total extract |
|---|---|---|---|---|
| | Calenduladiol | 0.051± 0.009 | 0.0416± 0.003 | 0.0590± 0.003 |
| | Arnidiol | 0.135± 0.01 | 0.0978± 0.009 | 0.131± 0.006 |
| | Faradiol | 0.750± 0.06 | 0.594± 0.002 | 0.758 ± 0.016 |
| MYRISTATES | Calenduladiol | 0.363± 0.010 | 0.248± 0.009 | 0.293± 0.010 |
| | Arnidiol | 0.876± 0.010 | 0.683± 0.009 | 0.809± 0.007 |
| | Faradiol | 3.122±0.092 | 2.086± 0.011 | 2.525± 0.014 |
| PALMITATES | Calenduladiol | 0.196± 0.010 | 0.121± 0.009 | 0.128 ± 0.009 |
| | Arnidiol | 0.516± 0.042 | 0.364± 0.010 | 0.448± 0.010 |
| | Faradiol | 2.013± 0.091 | 1.164± 0.010 | 1.285± 0.040 |
| % TOTAL | | 8.02 ± 0.091 | 5.40 ± 0.010 | 6.44 ± 0.013 |

From these results, it is inferred that the compounds most present were the faradiol esters with myristic and palmitic acid.

The following table reports the percentage content of each triterpene ester with respect to the total triterpene esters in extracts A and B.

| | | Extract A: % of each triterpene ester with respect to the total triterpene esters | Extract B: % of each triterpene ester with respect to the total triterpene esters |
|---|---|---|---|
| LAURATES | Calenduladiol | 0.64 | 0.77 |
| | Arnidiol | 1.68 | 1.81 |
| | Faradiol | 9.35 | 11.0 |
| MYRISTATES | Calenduladiol | 4.53 | 4.59 |
| | Arnidiol | 10.9 | 12.6 |
| | Faradiol | 38.9 | 38.6 |
| PALMITATES | Calenduladiol | 2.44 | 2.24 |
| | Arnidiol | 6.434 | 6.74 |
| | Faradiol | 25.1 | 21.59 |

The analyses carried out for the determination of pollutant substances showed a substantial absence of pollutant products (heavy metals, pesticides and aflatoxins).

### Evaluation of the phytotherapy activity

In order to evaluate in an objective and reproducible manner the effectiveness of the total extract according to the invention and of the products that contain it, an *in vitro* effectiveness study was conducted at the Vitroscreen institute. This study made use of cosmetogenomic techniques and utilized reconstructed human tissues as model, in particular the "Full-thickness (FT) skin" model described in detail below. Such models are considered reproducible, predictive and sensitive for evaluating the safety of ingredients and products and for investigating the action mechanism of the irritating substances (ECVAM -Atla 33 Suppl 1,47-81 -2005).

They are also considered valid study models in skin biology research, since they have the same multilayered morphology and the same functionality of the *in vivo* tissues. In addition, the absorption of topically-applied products is modulated by the presence of the barrier function, and hence it accounts for the penetration into the corneous layer.

### Assay on in vitro reconstructed human skin

The evaluation of the anti-inflammatory and cicatrizing effectiveness was carried out by using the "Full-thickness (FT) skin" (Phenion GmbH &Co, Frankfurt am Main, Germany) model as biological system, in an inflammatory lesion experimental protocol set up by Vitroscreen. The FT-skin model is constituted by multilayered human skin, reconstructed *in vitro* by keratinocytes and fibroblasts deriving from the same donor. After a five-week cultivation period, the "full thickness" is completely developed and comprises epidermis, basal membrane and dermis. The model was characterized with regard to the expression of fundamental markers of differentiation at the epidermal level (cytokeratin 10, filaggrin, transglutaminase and involucrin), at the dermal-epidermal junctions (laminin and collagen IV) and at the dermal level (elastin fibronectin).

FT tissue models were used for the assay with 1.3 cm diameter, placed in a suitable quantity of ALI medium.

The following were examined in the assay:
▪ the extract prepared according to example 1, at 0.5% in vaseline olio;
▪ the lipogel prepared according to example 2;
▪ the oleolite prepared according to example 3.

As negative control, untreated FT-skin was used, while untreated and injured FT-skin was used as positive control. As reference substance, Kenacort® (triamcinolone acetate) was used.

The tissues were injured by making four symmetric incisions via a biopsy punch of 2 mm diameter, and they were immediately treated with the substances under examination for 6 h, 48 h and 72 h. The substances were separately applied on the skin surface by means of micropipette, in 50 µl amounts.

At the end of incubation, the tissues were rinsed with physiological solution, homogenized and placed in 300 µl lysis buffer for the RNA extraction and for subsequent real-time quantitative PCR.

The RNA extraction was carried out by using RNAqueous® kit (Applied Biosystems), which allows the quick isolation of the RNA on filter.

The RNA concentration was determined by reading the absorbance at 260 nm and 280 nm on a plate with 96 UV-transparent wells, using the following formula: A260 * dilution factor * 40 = µg RNA / ml .

After extraction, the RNA was retro-transcripted into cDNA, which was diluted and preserved at -20°C.

The real-time PCR on each sample was separately carried out with an Applied Biosystems 7500 thermocycler with the following program: 95°C 10 minutes / 40 cycles (95°C 15 seconds, 60°C 1 minute), using a TaqMan® plate (TaqMan® Array Plate), containing 32 genes specific for inflammation and cicatrization; in the relative quantification, an endogenous control was used as active reference for normalizing the amount of target cDNA.

The quantification of cDNA was conducted by using the TaqMan® fluorescent probes (Applied Biosystems).

During data acquisition, an over-expression or an under-regulation of twice the control value was considered significant.

### Results and discussion

The effectiveness of the products was evaluated by observing the expression of twenty-one genes involved in the different epidermal repair phases (acute phase, proliferative phase and remodeling phase).

### In particular:

The inflammatory response and the reepithelialization processes that occur during the acute phase were evaluated by observing the expression of the genes which code for the following proteins: IL-1α, IL-8, TNFα, IFNA1, HSP70, MAP3K8, NF-kFB, CD68, MMP2, MMP9, ADAM15, ADAMTS8;

The cellular adhesion, which occurs during the proliferative phase, was evaluated by observing the expression of the genes which code for the following proteins: ITGA1, ITGB2 and RPSA = LAMR1;

The reorganization of the cytoskeleton and the effects on cellular motility were evaluated by observing the expression of the genes which code for the following proteins: VEGF-C, CTNNB1, DNAI1, FLNB, BPI.

Reported in the following tables 4-6, for each assayed product, is the effect on gene expression: the + sign indicates that the product had a positive influence, i.e. favoring the cicatrizing process, in accordance with the specific mechanism regulated by the gene itself.

**Table 4 - Effect of the oleolite prepared according to example 3**

| Gene expressed | Acute phase | Proliferative phase | Remodeling phase |
|---|---|---|---|
| IL-1 | | + | + |
| IL-8 | + | + | |
| TNF-α | | + | |
| IFNA1 | + | | |
| HSP70 | + | | |
| MAP3K8 | + | + | |
| NF-kB | + | + | |
| MMP2 | | + | + |
| MMP9 | | + | + |
| ADAM15 | | + | |
| ITGA1 | | + | + |
| ITGB2 | | + | + |
| RPSA = LAMR1 | | + | |
| VEGF-C | | + | + |
| CTNNB1 | | + | |
| TPM2 | | + | |
| BPI | + | + | |

Applied on the injured skin surface, the oleolite of example 3 determines:
▪ an increase of the expression of IL-1α and IL-8 by the epithelial cells, stimulating their proliferation and migration during the proliferative and remodeling phases;
▪ an increase of TNF-α, which facilitates the reepithelialization process;
▪ an increase of interferon alpha (IFNA1), which is involved in the organism defense processes and in acute phase induces the penetration of dendritic cells into the wound;
▪ an early over-expression of HSP70, a thermal shock protein which is expressed in adapting to stress situations;
▪ initial increase of MAP3K8, which promotes the production of TNF alpha and the activation of NF-kB, a transcription factor which has a protective role, limiting the inflammatory and fibrogenic response;
▪ over-expression of the metalloproteinases MMP2-MMP9, which facilitate the migration of the epithelial cells to the injury area;
▪ over-expression of ADAM15, which facilitates the cicatrization by increasing the bond with integrins, the migration of the keratinocytes and the remodeling of the matrix; increase of the integrins, which determines changes in the mechanical force of the cytoskeleton facilitating cellular motility and cicatrization;
▪ increase of LAMR1 (laminin receptor), which facilitates the reepithelialization;
▪ over-expression of VEGF-C by the fibroblasts, which accelerates the closure of the wound;
▪ activation of catenin and tropomyosin, proteins of the cytoskeleton which actively participate in the proliferative phase of the cicatrization.

The oleolite according to example 3 also has an anti-bacterial action, since it stimulates the production of BPI (bactericidal/permeability-increasing protein) by dermal fibroblasts, helping to oppose local inflammation.

**Table 5 - Effect of the total extract prepared according to example 1 at 0.5% in vaseline oil**

| Gene expressed | Acute phase | Proliferative phase | Remodeling phase |
|---|---|---|---|
| IL-1 | | + | + |
| IL-8 | + | + | |
| TNF-α | | + | |
| IFNA1 | + | | |
| HSP70 | + | | |
| MAP3K8 | + | + | |
| NF-kB | + | + | |
| MMP2 | | + | + |
| MMP9 | | + | + |
| ADAMTS8 | | + | |
| ITGA1 | | + | + |
| ITGB2 | | + | + |
| VEGF-C | | + | + |
| DNAI1 | | + | |
| FLNB | | + | |
| TPM2 | | + | |
| BPI | + | + | |

Like the oleolite of example 3, the total extract of example 1 is particularly effective in the proliferative phase, but to a certain extent it also affects the remodeling phase; it also carries out a certain anti-bacterial activity.

The extract of example 1 differs from the oleolite of example 3 in that it has greater cicatrizing effectiveness; this is due to the better contraction of the epidermal and dermal fibers, ascribable to a greater expression of ADAMTS8, dynein (DNAI1) and filamin beta (FLNB).

**Table 6 - Effect of the lipogel prepared according to example 2**

| Gene expressed | Acute phase | Proliferative phase | Remodeling phase |
|---|---|---|---|
| IL-1 | | + | + |
| IL-8 | | + | |
| TNF-α | | + | |
| CD68 | | + | |
| MMP9 | | + | + |
| ITGB2 | | + | + |
| RPSA = LAMR1 | | + | |
| VEGF-C | | + | + |
| CTNNB1 | | + | |
| DNAI1 | | + | |

From the above, it is observed that the lipogel acts in the proliferative phase, while it has a limited action in the remodeling phase. Analogous to the oleolite of example 3 and to the total extract of example 2, the lipogel causes the increase of expression of IL-1, IL-8, TNF-α, MMP9, ITGB2, RPSA = LAMR1 and VEGF-C; with the oleolite of example 3, it shares the capacity to increase the production of CTNNB1, while with the extract of example 2, it shares the capacity to increase the expression of DNAI1.

The lipogel does not have anti-bacterial activity, since it does not cause an over-expression of BPI. In addition, it was observed that the lipogel stimulates the expression of CD68; present in the lysosomes and endosomes and at the cellular surface, this functions as a scavenger molecule, mediating the recruitment and activation of the macrophages.

**Table 4 - Effect of the Kenacort®**

| Gene expressed | Acute phase | Proliferative phase | Remodeling phase |
|---|---|---|---|
| TNF-α | | + | |
| IFNA1 | + | | |
| MMP9 | | + | + |
| ITGA1 | | + | + |
| CTNNB1 | | + | |
| DNAI1 | | + | |
| FLNB | | + | |
| TPM2 | | + | |

The Kenacort®, used as a reference product, has no activity on the interleukin production (the expression level of IL8 is equivalent to that observed on injured, untreated tissue), thus slowing the migration of the epithelial cells to the injured site, a characteristic effect of the *in vivo* cortisone derivatives. It also increases the expression of MMP9, but not that of the ADAM proteins, slowing the cicatrization. Finally, it acts on all the proteins of the cytoskeleton, inducing a general, late reorganization thereof, exactly as occurs *in vivo.*

## Claims

1. A process for extracting flowering tops of *Calendula officinalis* comprising the following stages:
a) providing flowering tops of *Calendula officinalis*;
b) subjecting said flowering tops to extraction with carbon dioxide in supercritical phase at a temperature comprised between 60 and 70°C and at a pressure comprised between 600 and 700 bar, for a time period comprised between 200 and 250 min;
c) decreasing the pressure to 200-300 bar and collecting the extract A thus obtained;
d) further decreasing the pressure to 60-70 bar and collecting the extract B thus obtained.

2. Process according to claim 1), wherein the stage b) is carried out for 230 min.

3. Process for preparing a total extract of flowering tops of *Calendula officinalis* comprising the mixing of the extracts A and B obtainable according to the process of claim 1 or 2, stages c) and d), in a weight ratio comprised between 0.5:9.5 and 3:7.

4. Process according to claim 3, wherein the weight ratio is 1:9.

5. Extracts of flowering tops of *Calendula officinalis* obtainable according to the process of claim 1) or 2), stages c) or d).

6. Total extract of flowering tops of *Calendula officinalis* obtainable according to the process of claims 3) or 4).

7. Total extract of flowering tops according to claim 6) for use as a medicament, wherein said medicament is in the form of a topical formulation containing said total extract in an amount comprised between 0.01 and 6% by weight with respect to the overall weight of said formulation

8. Topical pharmaceutical or cosmetic formulation containing the extract of claim 6) in a mixture with suitable excipients and/or carriers, wherein said total extract is contained in said topical pharmaceutical or cosmetic composition in an amount comprised between 0.01 and 6% by weight with respect to the overall weight of said formulation.

9. Use of the total extract of flowering tops according to claim 6) in cosmetic formulations, wherein said total extract is contained in said topical cosmetic formulation in an amount comprised between 0.01 and 6% by weight with respect to the overall weight of said formulation .

## Patentansprüche

1. Verfahren zum Extrahieren von blühenden Spitzen von *Calendula officinalis,* umfassend die folgenden Stufen:
a) Bereitstellung von blühenden Spitzen von *Calendula officinalis;*
b) Unterwerfung der genannten blühenden Spitzen an eine Extraktion mit Kohlendioxid in einer überkritischen Phase bei einer Temperatur zwischen 60°C und 70°C und einem Druck zwischen 600 und 700 bar, für einen Zeitraum zwischen 200 und 250 min;
c) Verminderung des Druckes auf 200 bis 300 bar und Sammlung des so erhaltenen Extraktes A;
d) weitere Verminderung des Druckes auf 60 bis 70 bar und Sammlung des so erhaltenen Extraktes B.

2. Verfahren nach Anspruch 1, wobei die Stufe b) für 230 min durchgeführt wird.

3. Verfahren zur Herstellung eines Gesamtextraktes von blühenden Spitzen von *Calendula officinalis,* umfassend das Vermischen der nach dem Verfahren von Anspruch 1 oder 2 erhältlichen Extrakte A und B, Stufe c) und d), in einem Gewichtsverhältnis zwischen 0,5:95 und 3:7.

4. Verfahren nach Anspruch 3, wobei das Gewichtsverhältnis 1:9 ist.

5. Extrakte von blühenden Spitzen von *Calendula officinalis,* die nach dem Verfahren von Anspruch 1 oder 2, Stufen c) oder d), erhältlich sind.

6. Gesamtextrakt von blühenden Spitzen von *Calendula officinalis,* der nach dem Verfahren von Anspruch 3 oder 4 erhältlich ist.

7. Gesamtextrakt von blühenden Spitzen nach Anspruch 6 zur Verwendung als Medikament, wobei das Medikament in Form einer topischen Formulierung vorliegt, die den Gesamtextrakt in einer Menge zwischen 0,01 und 6 Gew.-%, bezogen auf das Gesamtgewicht der genannten Formulierung enthält.

8. Topische pharmazeutische oder kosmetische Formulierung, die den Extrakt nach Anspruch 6 in einer Mischung mit geeigneten Exzipienten und/oder Trägern enthält, wobei der Gesamtextrakt in der topischen pharmazeutischen oder kosmetischen Zusammensetzung in einer Menge zwischen 0,01 und 6 Gew.-% bezogen auf das Gesamtgewicht der Formulierung enthalten ist.

9. Verwendung des Gesamtextraktes von blühenden Spitzen nach Anspruch 6 in kosmetischen Formulierungen, wobei der Gesamtextrakt in der topischen kosmetischen Formulierung in einer Menge zwischen 0,01 und 6 Gew.-%, bezogen auf das Gesamtgewicht der Formulierung enthalten ist.

## Revendications

1. Procédé d'extraction de sommités fleuries de *Calendula officinalis* comprenant les étapes suivantes :
a) fournir des sommités fleuries de *Calendula officinalis* ;
b) soumettre lesdites sommités fleuries à une extraction avec du dioxyde de carbone en phase supercritique à une température comprise entre 60°C et 70°C et à une pression comprise entre 600 et 700 bars pour une période comprise entre 200 et 250 min ;
c) réduire la pression à 200-300 bars et recueillir l'extrait A ainsi obtenu ;
d) réduire encore la pression à 60-70 bars et recueillir l'extrait B ainsi obtenu.

2. Procédé selon la revendication 1, dans lequel l'étape b) est réalisée pendant 230 min.

3. Procédé de préparation d'un extrait total de sommités fleuries de *Calendula officinalis* comprenant le mélange des extraits A et B obtenus selon le procédé de la revendication 1 ou 2, étapes c) et d), dans un rapport pondéral compris entre 0,5:9,5 et 3:7.

4. Procédé selon la revendication 3, dans lequel le rapport pondéral est de 1:9.

5. Extraits de sommités fleuries de *Calendula officinalis* obtenues selon le procédé de la revendication 1 ou 2, étapes c) ou d).

6. Extrait total de sommités fleuries de *Calendula officinalis* obtenues selon le procédé des revendications 3 ou 4.

7. Extrait total de sommités fleuries selon la revendication 6 destiné à être utilisé en tant que médicament, dans lequel ledit médicament est sous la forme d'une formulation topique contenant ledit extrait total en une quantité comprise entre 0,01 et 6% en poids par rapport au poids total de ladite formulation.

8. Formulation pharmaceutique ou cosmétique topique contenant l'extrait de la revendication 6 en mélange avec des excipients et/ou des supports appropriés, dans laquelle ledit extrait total est contenu dans ladite composition topique pharmaceutique ou cosmétique en une quantité comprise entre 0,01 et 6% en poids par rapport au poids total de ladite formulation.

9. Utilisation de l'extrait total de sommités fleuries selon la revendication 6 dans des formulations cosmétiques, dans lequel ledit extrait total est contenu dans ladite formulation cosmétique topique en une quantité comprise entre 0,01 et 6% en poids par rapport au poids total de ladite formulation.
